(19) Europäisches Patentamt
European Patent Office
Office européen des brevets

(11) Publication number: 0 273 011 B1

(12) **EUROPEAN PATENT SPECIFICATION**

(45) Date of publication of patent specification: **17.03.93**

(51) Int. Cl.5: **C07C 229/04**, C07C 219/04, C09K 15/20, C08K 5/17, C08K 5/32

(21) Application number: **87810764.8**

(22) Date of filing: **18.12.87**

(54) **N,N-bis(hydroxyethyl)hydroxylamine ester stabilizers.**

(30) Priority: **24.12.86 US 946222**

(43) Date of publication of application: **29.06.88 Bulletin 88/26**

(45) Publication of the grant of the patent: **17.03.93 Bulletin 93/11**

(84) Designated Contracting States: **DE FR GB IT NL SE**

(56) References cited:
**GB-A- 1 001 098**
**GB-A- 1 264 920**
**US-A- 4 277 319**

(73) Proprietor: **CIBA-GEIGY AG**
**Klybeckstrasse 141**
**CH-4002 Basel(CH)**

(72) Inventor: **Ravichandran, Ramanathan**
**111 DeHaven Drive No. 125**
**Yonkers New York 10703(US)**
Inventor: **Seltzer, Raymond**
**11 Angus Lane**
**New City New York 10956(US)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 273 011 B1

## Description

The present invention relates to novel N,N-bis(hydroxyethyl)hydroxylamine esters, to the organic material stabilized with the aid of said compounds and to the use thereof for stabilizing an organic material against oxidative, thermal and actinic degradation.

Organic polymeric materials such as plastics and resins are subject to thermal, oxidative and photodegradation. A great variety of stabilizers are known in the art for stabilizing a diversity of substrates. Their effectiveness varies depending upon the causes of degradation and the substrate stabilized. In general, it is difficult to predict which stabilizer will be most effective and most economical for any one area of application. For example, stabilizer effectiveness in reducing volatility may depend upon preventing bond scission in the substrate molecule. Limiting embrittlement and retaining elasticity in a polymer or rubber may require prevention of excessive crosslinking and/or chain scission. Prevention of discoloration may require inhibiting reactions which yield new chromophores or color bodies in the substrate or stabilizer. Problems of process stability and incompatibility must also be considered.

Various organic hydroxylamine compounds are generally known and some are commercially available. A number of patients disclose nitrogen-substituted hydroxylamines as antioxidant stabilizers for various substrates including polyolefins, polyesters and polyurethanes. U.S. 3,432,578, 3,644,278, 3,778,464, 3,408,422, 3,926,909, 4,316,996, 4,386,224 and 4,590,231 are representative of such patents which basically disclose N,N-dialkyl-, N,N-diaryl- and N,N-diaralkyl hydroxylamine compounds and their color improvement and color stabilizing activity. In addition, N-hydroxyimino acids and esters thereof are well known in the literature and have been indicated as biologically active. For example, N-hydroxyiminodiacetic acid is a commercially available compound.

Representative publications disclosing such compounds include Kneifel and Bayer, J. Am. Chem. Soc. 108, 3075-3077 (1986) which describes the stereochemistry and synthesis of a vanadium complex of N-(L-1-carboxyethyl)-N-hydroxy-L-alanine; Felcman et al, Inorg. Chem. Acta. 93, 101-108 (1984) which describes the synthesis and stability of several transition metal complexes of N-hydroxyiminodiacetic acid and the corresponding iminodipropionic acid; and Japan Kokai 83-120,250 which describes a silver halide color developing solution containing a pyrrolidone polymer and hydroxyiminodiacetic acid. Polymer stabilization utility for these compounds is not mentioned.

The present invention pertains to novel compounds of formula I

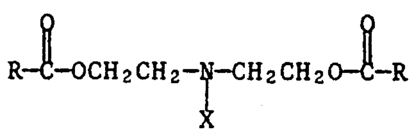

(I)

wherein each R is alkyl of 1 to 36 carbon atoms, cycloalkyl of 5 to 12 carbon atoms, phenyl, phenyl substituted by alkyl of 1 to 36 carbon atoms, aralkyl of 7 to 9 carbon atoms, said aralkyl substituted by alkyl of 1 to 36 carbon atoms, or is

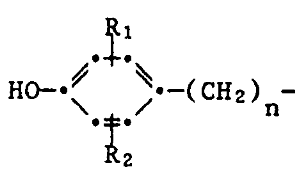

(II)

wherein $R_1$ and $R_2$ are independently alkyl of 1 to 18 carbon atoms, cycloalkyl of 5 to 12 carbon atoms, phenyl or aralkyl of 7 to 9 carbon atoms, and n is 0, 1 or 2; and X is hydroxyl or

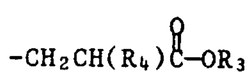

wherein $R_3$ is alkyl of 1 to 36 carbon atoms, cycloalkyl of 5 to 12 carbon atoms, phenyl, phenyl substituted by alkyl of 1 to 36 carbon atoms, aralkyl of 7 to 9 carbon atoms or said aralkyl substituted by alkyl of 1 to 36 carbon atoms, and $R_4$ is hydrogen or methyl.

The compounds of formula I exhibit a variety of desirable stabilizing properties. Thus, the compounds serve to protect various substrates such as polyolefins, elastomers and lubricating oils against the adverse effects of oxidative and thermal degradation. They are most effective as lubricant stabilizers and as color improvers and process stabilizers in polyolefin compositions which may contain metal salts of fatty acids and which also contain a phenolic antioxidant. Thus, they serve to substantially reduce color formation resulting from the presence of the phenolic antioxidant and/or from the processing conditions as well as to directly protect the polymer from said processing conditions. They also prevent the discoloration of polyolefin compositions contained hindered amine light stabilizers or combinations of phenolic antioxidants and organic phosphites. In addition, the gas fading that may be experienced upon exposure to the combustion products of natural gas is also significantly reduced.

Examples of R and $R_3$ as alkyl of 1 to 36 carbon atoms are methyl, n-butyl, tert-butyl, n-octyl, 2-ethylhexyl, decyl, dodecyl, octadecyl, eicosyl, docosyl, pentacosyl, heptacosyl, triacontyl, dotriacontyl, tetratriacontyl and hexatriacontyl. Straight-chain or branched alkyl of 1 to 18 carbon atoms is preferred.

R, $R_1$, $R_2$ and $R_3$ as cycloalkyl of 5 to 12 carbon atoms are e.g. cyclopentyl, cyclohexyl, cycloheptyl, cyclooctyl, cyclodecyl or cyclododecyl. Cycloalkyl of 5 to 8 carbon atoms is preferred. Cyclopentyl and cyclohexyl are especially preferred.

R and $R_3$ as aralkyl of 7 to 9 carbon atoms or such aralkyl being substituted by alkyl of 1 to 36 carbon atoms may be in particular phenylalkyl of 7 to 9 carbon atoms where the phenyl ring is optionally substituted by alkyl of 1 to 36 carbon atoms. Examples are benzyl, α-methylbenzyl, α,α-dimethylbenzyl, 3- or 4-methylbenzyl, 3,5-dimethylbenzyl and 4-nonylbenzyl. Benzyl, α-methylbenzyl and α,α-dimethylbenzyl are preferred.

$R_1$ and $R_2$ as aralkyl of 7 to 9 carbon atoms are for example phenylalkyl of 7 to 9 carbon atoms, in particular benzyl.

$R_1$ and $R_2$ as alkyl of 1 to 18 carbon atoms are for example methyl, n-butyl, tert-butyl, n-octyl, 2-ethylhexyl, decyl, dodecyl or octadecyl. Alkyl of 4 to 8 carbon atoms, in particular tert-butyl, is preferred.

Those compounds of formula I are preferred, wherein the radicals R are independently alkyl of 1 to 18 carbon atoms, cyclopentyl, cyclohexyl, phenyl, benzyl, α-methylbenzyl or α,α-dimethylbenzyl.

R is preferably a group of the formula II, in particular a group of the formula IIa

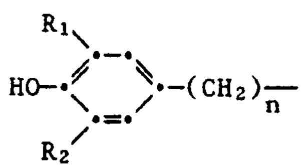

(IIa) .

According to a further preferred embodiment X is hydroxyl or

$$-CH_2CH(R_4)-\overset{O}{\overset{\|}{C}}-OR_3,$$

in particular

$$-CH_2CH_2-\overset{O}{\overset{\|}{C}}-O-(C_1-C_{18}\text{-alkyl}).$$

Preferred compounds of formula I are
N,N-bis[2-stearoyloxyethyl]-N-[2′-methoxycarbonylethyl]amine,
N,N-bis[2-stearoyloxyethyl]hydroxylamine,
N,N-bis[2-acetoxyethyl]-N-[2′-methoxycarbonylethyl]amine,
N,N-bis[2-acetoxyethyl]hydroxylamine,
N,N-bis[2-(3′,5′-di-tert-butyl-4′-hydroxyphenylpropionyloxy)ethyl]-N-[2″-methoxycarbonylethyl]amine,
N,N-bis[2-(3′,5′-di-tert-butyl-4′-hydroxyphenylpropionyloxy)ethyl]hydroxylamine,

N,N-bis[2-(3′,5′-di-tert-butyl-4′-hydroxybenzoyloxy)ethyl]-N-[2″-methoxycarbonylethyl]amine and N,N-bis[2-(3′,5′-di-tert-butyl-4′-hydroxybenzoyloxy)ethyl]hydroxylamine.

The compounds of formula I can be prepared by methods known per se, e.g. by (1) reacting diethanolamine with the appropriately substituted acrylate or methacrylate of the formula $CH_2=C(R_4)-COOR_3$ at a temperature usually ranging from 25 to 200 ° C to prepare the N-substituted diethanolamine starting product and (2) reacting said N-substituted diethanolamine with RCOCl in a solvent such as e.g. methylene chloride and in the presence of a proton acceptor, for example a tertiary amine, in particular triethylamine or pyridine, at a temperature preferably ranging from 25 to 80 ° C. The resulting product reflects the compound of formula I wherein X is

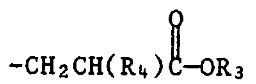 .

In order to prepare the hydroxylamine product, i.e. X = -OH, the product resulting from step (2) is conveniently reacted with an oxidizing agent such as e.g. m-chloroperoxybenzoic acid, in a solvent, preferably methylene chloride at a temperature usually ranging from 0 to 35 ° C.

The various reactants are commercially available or can be prepared by known methods. For example, the latter formation of the hydroxylamine derivative proceeds according to the reaction described in Cope et al, Organic Reactions 11, Chapter 5, 317 (1960).

A further embodiment of the invention is a composition comprising an organic material subject to oxidative, thermal and actinic degradation and at least one compound of formula I.

The compounds of formula I are effective in stabilizing organic materials e.g. plastics, polymers and resins in addition to mineral and synthetic fluids such as e.g. lubricating oils, circulating oils, etc.

Substrates in which the compounds of formula I are particularly useful are polyolefin homo- and copolymers such as e.g. polyethylene and polypropylene, polystyrene, including impact polystyrene, ABS resin, styrene/butadiene rubber, styrene/butadiene block copolymer, isoprene, as well as natural rubber, polyesters including polyethylene terephthalate and polybutylene terephthalate, including copolymers, polyphenylene oxide and lubricating oils such as those derived from mineral oil.

In general polymers which can be stabilized include:

1. Polymers of monoolefins and diolefins, for example polypropylene, polyisobutylene, polybutene-1, polymethylpentene-1, polyisoprene or polybutadiene, as well as polymers of cycloolefins, for instance of cyclopentene or norbornene, polyethylene (which optionally can be crosslinked), for example high density polyethylene (HDPE), low density polyethylene (LDPE and linear low density polyethylene (LLDPE).

2. Mixtures of the polymers mentioned under 1), for example mixtures of polypropylene with polyisobutylene, polypropylene with polyethylene (for example PP/HDPE, PP/LDPE) and mixtures of different types of polyethylene (for example LDPE/HDPE).

3. Copolymers of monoolefines and diolefines with each other or with other vinyl monomers, such as, for example, ethylene/propylene, linear low density polyethylene (LLDPE) and its mixtures with low density polyethylene (LDPE), propylene/butene-1, ethylene/hexene, ethylene/ethylpentene, ethylene/heptene, ethylene/octene, propylene/isobutylene, ethylene/butene-1, propylene/butadiene, isobutylene/isoprene, ethylene/alkyl acrylates, ethylene/alkyl methacrylates, ethylene/vinyl acetate or ethylene/ acrylic acid copolymers and their salts (ionomers) and terpolymers of ethylene with propylene and a diene, such as hexadiene, dicyclopentadiene or ethylidene-norbornene; as well as mixtures of such copolymers and their mixtures with polymers mentioned in 1) above, for example polypropylene/ethylene-propylene-copolymers, LDPE/EVA, LDPE/EAA, LLDPE/EVA and LLDPE/EAA.

3a. Hydrocarbon resins (for example $C_5$-$C_9$) and hydrogenated modifications thereof (for example tackyfiers).

4. Polystyrene, poly-(p-methylstyrene), poly-(α-methylstyrene).

5. Copolymers of styrene or α-methylstyrene with dienes or acrylic derivatives, such as, for example, styrene/butadiene, stryene/acrylonitrile, styrene/alkyl methacrylate, styrene/maleic anhydride, styrene/butadiene/ethyl acrylate, styrene/acrylonitrile/methyl acrylate; mixtures of high impact strength from styrene copolymers and another polymer, such as, for example, from a polyacrylate, a diene polymer or an ethylene/propylene/diene terpolymer; and block copolymers of styrene, such as, for

example, styrene/butadiene/styrene, styrene/ isoprene/styrene, styrene/ethylene/butylene/styrene or styrene/ ethylene/propylene/styrene.

6. Graft copolymers of styrene or α-methylstyrene such as, for example, styrene on polybutadiene, styrene on polybutadiene-styrene or polybutadiene-acrylonitrile; styrene and acrylonitrile (or methacrylonitrile) on polybutadiene; styrene and maleic anhydride or maleimide on polybutadiene; styrene, acrylonitrile and maleic anhydride or maleimide on polybutadiene; styrene, acrylonitrile and methyl methacrylate on polybutadiene, styrene and alkyl acrylates or methacrylates on polybutadiene, styrene and acrylonitrile on ethylene/propylene/diene terpolymers, styrene and acrylonitrile on polyacrylates or polymethacrylates, styrene and acrylonitrile on acrylate/butadiene copolymers, as well as mixtures thereof with the copolymers listed under 5), for instance the copolymer mixtures known as ABS-, MBS-, ASA- or AES-polymers.

7. Halogen-containing polymers, such as polychloroprene, chlorinated rubbers, chlorinated or sulfochlorinated polyethylene, epichlorohydrine homo- and copolymers, polymers from halogen-containing vinyl compounds, as for example, polyvinylchloride, polyvinylidene chloride, polyvinyl fluoride, polyvinylidene fluoride, as well as copolymers thereof, as for example, vinyl chloride/vinylidene chloride, vinyl chloride/vinyl acetate or vinylidene chloride/vinyl acetate copolymers.

8. Polymers which are derived from α,β-unsaturated acids and derivatives thereof, such as polyacrylates and polymethacrylates, polyacrylamide and polyacrylonitrile.

9. Copolymers from the monomers mentioned under 8) with each other or with other unsaturated monomers, such as, for instance, acrylonitrile/butadien, acrylonitrile/alkyl acrylate, acrylonitrile/alkoxyalkyl acrylate or acrylonitrile/vinyl halogenide copolymers or acrylonitrile/alkyl methacrylate/butadiene terpolymers.

10. Polymers which are derived from unsaturated alcohols and amines, or acyl derivatives thereof or acetals thereof, such as polyvinyl alcohol, polyvinyl acetate, polyvinyl stearate, polyvinyl benzoate, polyvinyl maleate, polyvinylbutyral, polyallyl phthalate or polyallyl-melamine; as well as their copolymers with olefins mentioned in 1) above.

11. Homopolymers and copolymers of cyclic ethers, such as polyalkylene glycols, polyethylene oxide, polypropylene oxide or copolymers thereof with bis-glycidyl ethers.

12. Polyacetals, such as polyoxymethylene and those polyoxymethylenes which contain ethylene oxide as a comonomer; polyacetals modified with thermoplastic polyurethanes, acrylates or MBS.

13. Polyphenylene oxides and sulfides, and mixtures of polyphenylene oxides with polystyrene or polyamides.

14. Polyurethanes which are derived from polyethers, polyesters or polybutadiens with terminal hydroxyl groups on the one side and aliphatic or aromatic polyisocyanates on the other side, as well as precursors thereof (polyisocyanates, polyols or prepolymers).

15. Polyamides and copolyamides which are derived from diamines and dicarboxylic acids and/or from aminocarboxylic acids or the corresponding lactams, such as polyamide 4, polyamide 6, polyamide 6/6, 6/10, 6/9, 6/12 and 4/6, polyamide 11, polyamide 12, aromatic polyamides obtained by condensation of m-xylene, diamine and adipic acid; polyamides prepared from hexamethylene diamine and isophthalic or/and terephthalic acid and optionally an elastomer as modifier, for example poly-2,4,4,-trimethylhexamethylene terephthalamide or poly-m-phenylene isophthalamide. Further copolymers of the aforementioned polyamides with polyolefins, olefin copolymers, ionomers or chemically bonded or grafted elastomers; or with polyethers, such as for instance, with polyethylene glycol, polypropylene glycol or polytetramethylene glycols. Polyamides or copolyamides modified with EPDM or ABS. Polyamides condensed during processing (RIM-polyamide systems).

16. Polyureas, polyimides and polyamide-imides.

17. Polyesters which are derived from dicarboxylic acids and diols and/or from hydroxycarboxylic acids or the corresponding lactones, such as polyethylene terephthalate, polybutylene terephthalate, poly-1,4-dimethylol-cyclohexane terephthalate, poly-[2,2,-(4-hydroxyphenyl)-propane] terephthalate and polyhydroxybenzoates as well as block-copolyether-esters derived from polyethers having hydroxyl end groups.

18. Polycarbonates and polyester-carbonates.

19. Polysulfones, polyethersulfones and polyetherketones.

20. Crosslinked polymers which are derived from aldehydes on the one hand and phenols, ureas and melamines on the other hand, such as phenol/formaldehyde resins, urea/formaldehyde resins and melamine/formaldehyde resins.

21. Drying and non-drying alkyd resins.

22. Unsaturated polyester resins which are derived from copolyesters of saturated and unsaturated dicarboxylic acids with polyhydric alcohols and vinyl compounds as crosslinking agents, and also halogen-containing modifications thereof of low inflammability.

23. Thermosetting acrylic resins, derived from substituted acrylic esters, such as epoxy-acrylates, urethane-acrylates or polyester-acrylates.

24. Alkyd resins, polyester resins or acrylate resins in admixture with melamine resins, urea resins, polyisocyanates or epoxide resins as crosslinking agents.

25. Crosslinked epoxide resins which are derived from polyepoxides, for example from bis-glycidyl ethers or from cycloaliphatic diepoxides.

26. Natural polymers, such as cellulose, rubber, gelatine and derivatives thereof which are chemically modified in a polymerhomologous manner, such as cellulose acetates, cellulose propionates and cellulose butyrates, or the cellulose ethers, such as methylcellulose; rosins and their derivatives.

27. Mixtures of polymers as mentioned above, for example PP/EPDM, Polyamide 6/EPDM or ABS, PVC/EVA, PVC/ABS, PVC/MBS, PC/ABS, PBTP/ABS, PC/ASA, PC/PBT, PVC/CPE, PVC/acrylates, POM/thermoplastic PUR, PC/thermoplastic PUR, POM/acrylate, POM/MBS, PPE/HIPS, PPE/PA 6.6 and copolymers, PA/HDPE, PA/PP, PA/PPE.

28. Naturally occurring and synthetic organic materials which are pure monomeric compounds or mixtures of such compounds, for example mineral oils, animal and vegetable fats, oil and waxes, or oils, fats and waxes based on synthetic esters (e.g. phthalates, adipates, phosphates or trimellithates) and also mixtures of synthetic esters with mineral oils in any weight ratios, which materials may be used as plasticizer for polymers or as textile spinning oils, as well as aqueous emulsions of such materials.

29. Aqueous emulsions of natural or synthetic rubber, e.g. natural latex or latices or carboxylated styrene/butadiene copolymers.

Preferred compositions are those, wherein the organic material is a synthetic polymer, in particular a polyolefin homopolymer or copolymer.

In general, the compounds of formula I are employed e.g. in from about 0.01 to about 5 % by weight of the stabilized composition, although this will vary with the particular substrate and application. An advantageous range is from about 0.5 to about 2 %, and especially about 0.1 to about 1 %.

The compounds of formula I may readily be incorporated into the organic material by conventional techniques, at any convenient stage prior to the manufacture of shaped articles therefrom. For example, the stabilizer may be mixed with the material to be stabilized in dry powder form, or a suspension or emulsion of the stabilizer may be mixed with a solution, suspension, or emulsion of the organic material. The resulting stabilized polymer compositions of the invention may optionally also contain various conventional additives, such as the following:

1. Antioxidants

1.1. Alkylated monophenols, for example 2,6-di-tert-butyl-4-methylphenol, 2-tert-butyl-4,6-dimethylphenol, 2,6-di-tert-butyl-4-ethylphenol, 2,6-di-tert-butyl-4-n-butylphenol, 2,6-di-tert-butyl-4-isobutylphenol, 2,6-dicyclopentyl-4-methylphenol, 2-($\alpha$-methylcyclohexyl)-4,6-dimethylphenol, 2,6-dioctadecyl-4-methylphenol, 2,4,6-tricyclohexylphenol, 2,6-di-tert-butyl-4-methoxymethylphenol, 2,6-di-nonyl-4-methylphenol.

1.2. Alkylated hydroquinones,for example 2,6-di-tert-butyl-4-methoxyphenol, 2,5-di-tert-butylhydroquinone, 2,5-di-tert-amylhydroquinone, 2,6-diphenyl-4-octadecyloxyphenol.

1.3. Hydroxylated thiodiphenyl ethers, for example 2,2′-thiobis(6-tert-butyl-4-methylphenol), 2,2′-thiobis(4-octylphenol), 4,4′-thiobis(6-tert-butyl-3-methylphenol), 4,4′-thiobis(6-tert-butyl-2-methylphenol).

1.4. Alkylidenebisphenols, for example 2,2′-methylenebis(6-tert-butyl-4-methylphenol), 2,2′-methylenebis-(6-tert-butyl-4-ethylphenol), 2,2′-methylenebis[4-methyl-6-($\alpha$-methylcyclohexyl)phenol], 2,2′-methylenebis-(4-methyl-6-cyclohexylphenol), 2,2′-methylenebis(6-nonyl-4-methylphenol), 2,2′-methylenebis(4,6-di-tert-butylphenol), 2,2′-ethylidenebis(4,6-di-tert-butylphenol), 2,2′-ethylidenebis(6-tert-butyl-4-isobutylphenol), 2,2′-methylenebis[6-($\alpha$-methylbenzyl)-4-nonylphenol], 2,2′-methylenebis[6-($\alpha$,$\alpha$-dimethylbenzyl)-4-nonylphenol], 4,4′-methylenebis(2,6-di-tert-butylphenol), 4,4′-methylenebis(6-tert-butyl-2-methylphenol), 1,1-bis(5-tert-butyl-4-hydroxy-2-methylphenyl)butane, 2,6-bis(3-tert-butyl-5-methyl-2-hydroxybenzyl)-4-methylphenol, 1,1,3-tris(5-tert-butyl-4-hydroxy-2-methylphenyl)butane, 1,1-bis(5-tert-butyl-4-hydroxy-2-methylphenyl)-3-n-dodecylmercaptobutane, ethylene glycol bis[3,3-bis(3′-tert-butyl-4′-hydroxyphenyl)-butyrate], bis(3-tert-butyl-4-hydroxy-5-methylphenyl)dicyclopentadiene, bis[2-(3′-tert-butyl-2′-hydroxy-5′-methylbenzyl)-6-tert-butyl-4-methylphenyl] terephthalate.

1.5. Benzyl compounds, for example 1,3,5-tris(3,5-di-tert-butyl-4-hydroxybenzyl)-2,4,6-trimethylbenzene, bis(3,5-di-tert-butyl-4-hydroxybenzyl) sulfide, isooctyl 3,5-di-tert-butyl-4-hydroxybenzylmercaptoacetate,

bis(4-tert-butyl-3-hydroxy-2,6-dimethylbenzyl) dithiolterephthalate, 1,3,5-tris(3,5-di-tert-butyl-4-hydroxybenzyl) isocyanurate, 1,3,5-tris(4-tert-butyl-3-hydroxy-2,6-dimethylbenzyl) isocyanurate, dioctadecyl 3,5-di-tert-butyl-4-hydroxybenzylphosphonate, calcium salt of monoethyl 3,5-di-tert-butyl-4-hydroxybenzylphosphonate, 1,3,5-tris(3,5-dicyclohexyl-4-hydroxybenzyl)isocyanurate.

1.6. Acylaminophenols, for example anilide of 4-hydroxylauric acid, anilide of 4-hydroxystearic acid, 2,4-bis(octylmercapto)-6-(3,5-di-tert-butyl-4-hydroxyanilino)-s-triazine, octyl N-(3,5-di-tert-butyl-4-hydroxyphenyl)carbamate.

1.7. Esters of β-(3,5-di-tert-butyl-4-hydroxyphenyl)propionic acid with mono- or polyhydric alcohols, e.g. with methanol, diethylene glycol, octadecanol, triethylene glycol, 1,6-hexanediol, pentaerythritol, neopentyl glycol, tris(hydroxyethyl) isocyanurate, thiodiethylene glycol, N,N′-bis(hydroxyethyl)oxalic acid diamide.

1.8. Esters of β-(5-tert-butyl-4-hydroxy-3-methylphenyl)propionic acid with mono- or polyhydric alcohols, e.g. with methanol, diethylene glycol, octadecanol, triethylene glycol, 1,6-hexanediol, pentaerythritol, neopentyl glycol, tris(hydroxyethyl) isocyanurate, thiodiethylene glycol, N,N′-bis(hydroxyethyl)oxalic acid diamide.

1.9. Esters of β-(3,5-dicyclohexyl-4-hydroxyphenyl)propionic acid with mono- or polyhydric alcohols, e.g. with methanol, diethylene glycol, octadecanol, triethylene glycol, 1,6-hexanediol, pentaerythritol, neopentyl glycol, tris(hydroxyethyl) isocyanurate, thiodiethylene glycol, N,N′-bis(hydroxyethyl)oxalic acid diamide.

1.10. Amides of β-(3,5-di-tert-butyl-4-hydroxyphenyl)propionic acid e.g. N,N′-bis(3,5-di-tert-butyl-4-hydroxyphenylpropionyl)hexamethylene-diamine, N,N′-bis(3,5-di-tert-butyl-4-hydroxyphenylpropionyl)-trimethylene-diamine, N,N′-bis(3,5-di-tert-butyl-4-hydroxyphenylpropionyl)hydrazine.

2. UV absorbers and light stabilisers

2.1. 2-(2′-Hydroxyphenyl)benzotriazoles, for example the 5′-methyl, 3′,5′-di-tert-butyl, 5′-tert-butyl, 5′-(1,1,3,3-tetramethylbutyl), 5-chloro-3′,5′-di-tert-butyl, 5-chloro-3′-tert-butyl-5′-methyl, 3′-sec-butyl-5′-tert-butyl, 4′-octoxy, 3′,5′-di-tert-amyl and 3′,5′-bis(α,α-dimethylbenzyl) derivatives.

2.2. 2-Hydroxybenzophenones, for example the 4-hydroxy, 4-methoxy, 4-octoxy, 4-decyloxy, 4-dodecyloxy, 4-benzyloxy, 4,2′,4′-trihydroxy and 2′-hydroxy-4,4′-dimethoxy derivatives.

2.3. Esters of substituted and unsubstituted benzoic acids, for example, 4-tert-butylphenyl salicylate, phenyl salicylate, octylphenyl salicylate, dibenzoylresorcinol, bis(4-tert-butylbenzoyl)resorcinol, benzoylresorcinol, 2,4-di-tert-butylphenyl 3,5-di-tert-butyl-4-hydroxybenzoate and hexadecyl 3,5-di-tert-butyl-4-hydroxybenzoate.

2.4. Acrylates, for example ethyl α-cyano-β,β-diphenylacrylate, isooctyl α-cyano-β,β-diphenylacrylate, methyl α-carbomethoxycinnamate, methyl α-cyano-β-methyl-p-methoxy-cinnamate, butyl α-cyano-β-methyl-p-methoxycinnamate, methyl α-carbomethoxy-p-methoxycinnamate and N-(β-carbomethoxy-β-cyanovinyl)-2-methylindoline.

2.5. Nickel compounds, for example nickel complexes of 2,2′-thiobis[4-(1,1,3,3-tetramethylbutyl)phenol], such as the 1:1 or 1:2 complex, with or without additional ligands such as n-butylamine, triethanolamine or N-cyclohexyldiethanolamine, nickel dibutyldithiocarbamate, nickel salts of 4-hydroxy-3,5-di-tert-butylbenzylphosphonic acid monalkyl esters, e.g. of the methyl or ethyl ester, nickel complexes of ketoximes, e.g. of 2-hydroxy-4-methylphenyl undecyl ketoneoxime, nickel complexes of 1-phenyl-4-lauroyl-5-hydroxypyrazole, with or without additional ligands.

2.6. Sterically hindered amines, for example bis(2,2,6,6-tetramethylpiperidyl) sebacate, bis(1,2,2,6,6-pentamethylpiperidyl) sebacate, bis(1,2,2,6,6-pentamethylpiperidyl) n-butyl-3,5-di-tert-butyl-4-hydroxybenzylmalonate, the condensation product of 1-hydroxyethyl-2,2,6,6-tetramethyl-4-hydroxypiperidine and succinic acid, the condensation product of N,N′-bis(2,2,6,6-tetramethyl-4-piperidyl)-hexamethylenediamine and 4-tert-octylamino-2,6-dichloro-1,3,5-s-triazine, tris(2,2,6,6-tetramethyl-4-piperidyl) nitrilotriacetate, tetrakis(2,2,6,6-tetramethyl-4-piperidyl)-1,2,3,4-butane tetracarboxylate, 1,1′-(1,2-ethanediyl)bis(3,3,5,5-tetramethylpiperazinone).

2.7. Oxalic acid diamides, for example 4,4′-dioctyloxyoxanilide, 2,2′-dioctyloxy-5,5′-di-tert-butyloxanilide, 2,2′-didodecyloxy-5,5′-di-tert-butyloxanilide, 2-ethoxy-2′-ethyloxanilide, N,N′-bis(3-dimethylaminopropyl)-oxalamide, 2-ethoxy-5-tert-butyl-2′-ethyloxanilide and its mixture with 2-ethoxy-2′-ethyl-5,4′-di-tert-butyloxanilide and mixtures of ortho- and para-methoxy-disubstituted oxanilides and mixtures of o- and p-ethoxy-disubstituted oxanilides.

3. Metal deactivators, for example N,N′-diphenyloxalic acid diamide, N-salicylal-N′-salicyloylhydrazine, N,N′-bis(salicyloyl)hydrazine, N,N′-bis(3,5-di-tert-butyl-4-hydroxyphenylpropionyl)hydrazine, 3-salicyloylamino-1,2,4-triazole, bis(benzylidene)oxalic acid dihydrazide.

4. Phosphites and phosphonites, for example triphenyl phosphite, diphenylalkyl phosphites, phenyldialkyl phosphites, tris(nonylphenyl) phosphite, trilauryl phosphite, trioctadecyl phosphite, distearyl pentaerythritol diphosphite, tris(2,4-di-tert-butylphenyl) phosphite, diisodecyl pentaerythritol diphosphite, bis(2,4-di-tert-butylphenyl) pentaerythritol diphosphite, tristearyl sorbitol triphosphite, tetrakis(2,4-di-tert-butylphenyl) 4,4′-biphenylene diphosphonite, 3,9-bis(2,4-di-tert-butylphenoxy)-2,4,8,10-tetraoxa-3,9-diphosphaspiro-[5.5]undecane.

5. Peroxide scavengers, for example esters of β-thiodipropionic acid, for example the lauryl, stearyl, myristyl or tridecyl esters, mercaptobenzimidazole or the zinc salt of 2-mercaptobenzimidazole, zinc dibutyldithiocarbamate, dioctadecyl disulfide, pentaerythritol tetrakis(β-dodecylmercapto)propionate.

6. Polyamide stabilisers, for example, copper salts in combination with iodides and/or phosphorus compounds and salts of divalent manganese.

7. Basic co-stabilisers, for example, melamine, polyvinylpyrrolidone, dicyandiamide, triallyl cyanurate, urea derivatives, hydrazine derivatives, amines, polyamides, polyurethanes, alkali metal salts and alkaline earth metal salts of higher fatty acids for example Ca stearate, Zn stearate, Mg stearate, Na ricinoleate and K palmitate, antimony pyrocatecholate or zinc pyrocatecholate.

8. Nucleating agents, for example, 4-tert.butyl-benzoic acid, adipic acid, diphenylacetic acid.

9. Fillers and reinforcing agents, for example, calcium carbonate, silicates, glass fibers, asbestos, talc, kaolin, mica, barium sulfate, metal oxides and hydroxydes, carbon black, graphite.

10. Other additives, for example, plasticisers, lubricants, emulsifiers, pigments, optical brighteners, flameproofing agents, antistatic agents and blowing agents.

The compositions of the invention preferably contain a metal salt of a higher fatty acid and/or a phenolic antioxidant as further additives.

While the compounds of formula I can be beneficially used as stabilizers for a variety of substrates, particularly the polyolefins, both alone and in conjunction with other coadditives, the introduction of the compounds of formula I into polyolefins, optionally containing various alkali metal, alkaline earth metal and aluminum salts of higher fatty acids (see Additive #7 hereinabove), with hindered phenolic antioxidants results in enhanced and particularly salubrious protection to such substrates in terms of reducing color formation stemming from the presence of the phenols. Such phenolic antioxidants include n-octadecyl 3,5-di-tert-butyl-4-hydroxyhydrocinnamate, neopentanetetrayl tetrakis(3,5-di-tert-butyl-4-hydroxyhydrocinnamate), di-n-octadecyl 3,5-di-tert-butyl-4-hydroxybenzylphosphonate, 1,3,5-tris(3,5-di-tert-butyl-4-hydroxybenzyl)isocyanurate, thiodiethylene bis(3,5-di-tert-butyl-4-hydroxyhydrocinnamate), 1,3,5-trimethyl-2,4,6-tris(3,5-di-tert-butyl-4-hydroxybenzyl)benzene, 3,6-dioxaoctamethylene bis(3-methyl-5-tert-butyl-4-hydroxyhydrocinnamate), 2,6-di-tert-butyl-p-cresol, 2,2′-ethylidene-bis(4,6-di-tert-butylphenol), 1,3,5-tris(2,6-dimethyl-4-tert-butyl-3-hydroxybenzyl)isocyanurate, 1,1,3-tris(2-methyl-4-hydroxy-5-tert-butylphenyl)butane, 1,3,5-tris[2-(3,5-di-tert-butyl-4-hydroxyhydrocinnamoyloxy)ethyl]isocyanurate, 3,5-bis(3,5-di-tert-butyl-4-hydroxybenzyl)mesitol, hexamethylene bis(3,5-di-tert-butyl-4-hydroxyhydrocinnamate), 1-(3,5-di-tert-butyl-4-hydroxyanilino)-3,5-bis(octylthio)-s-triazine , N,N′-hexamethylene-bis(3,5-di-tert-butyl-4-hydroxyhydrocinnamamide), calcium bis(ethyl-3,5-di-tert-butyl-4-hydroxybenzylphosphonate), ethylene bis[3,3-bis(3-tert-butyl-4-hydroxyphenyl)butyrate], octyl 3,5-di-tert-butyl-4-hydroxybenzylmercaptoacetate, bis(3,5-di-tert-butyl-4-hydroxyhydrocinnamoyl)hydrazide, and N,N′-bis[2-(3,5-di-tert-butyl-4-hydroxyhydrocinnamoyloxy)ethyl]oxamide.

Preferred antioxidants are neopentanetetrayl tetrakis(3,5-di-tert-butyl-4-hydroxyhydrocinnamate), n-octadecyl 3,5-di-tert-butyl-4-hydroxyhydrocinnamate, 1,3,5-trimethyl-2,4,6-tris(3,5-di-tert-butyl-4-hydroxybenzyl)benzene, 1,3,5-tris(3,5-di-tert-butyl-4-hydroxybenzyl)isocyanurate, 2,6-di-tert-butyl-p-cresol and 2,2′-ethylidene-bis(4,6-di-tert-butylphenol).

Likewise, the compounds of formula I prevent color formation when hindered amine light stabilizers are present, such hindered amines including bis(1,2,2,6,6-pentamethyl-4-piperidyl)-2-n-butyl-2-(3,5-di-tert-butyl-4-hydroxybenzyl)malonate; bis(2,2,6,6-tetramethyl-4-piperidyl)sebacate; dimethylsuccinate polymer with 1-hydroxyethyl-2,2,6,6-tetramethyl-4-hydroxypiperidine; and polymer of 2,4-dichloro-6-octylamino-s-triazine with N,N′-bis(2,2,6,6-tetramethyl-4-piperidyl)hexamethylenediamine.

The compositions of the invention preferably contain antioxidants, benzotriazole UV absorbers, hindered amine light stabilizers, phosphites, thiosynergists or mixtures thereof as further additives.

The following examples illustrate the embodiments of this invention. In these examples, all parts given are by weight unless otherwise specified.

Example 1: N-[2-Methoxycarbonylethyl]diethanolamine (starting product)

A mixture of 10.5 grams of diethanolamine and 17.2 grams of methyl acrylate is heated under reflux for 24 hours. The excess methyl acrylate is removed under reduced pressure and the residue is purified using flash chromatography to afford the title compound as a thick oil.

Example 2: N,N-Bis[2-stearoyloxyethyl]-N-[2′-methoxycarbonylethyl]amine

A solution of 6.7 grams of the compound of Example 1 in 50 ml of methylene chloride containing 9.8 ml of triethylamine is added dropwise to a solution of 21.22 grams of stearoyl chloride in 50 ml of methylene chloride, and the reaction mixture is stirred at room temperature for 12 hours. The precipitated triethylamine hydrochloride is removed by filtration and the filtrate is concentrated under reduced pressure. Purification of the residue by liquid chromatography affords the title compound as a white solid: m.p. 50 - 52 °C.
Anal. Calcd. for $C_{44}H_{85}NO_6$: C, 73.0; H, 11.8; N, 1.9.
Found: C, 73.0; H, 12.1; N, 1.9.

Example 3: N,N-Bis[2-stearoyloxyethyl]hydroxylamine

A solution of 6.0 grams of the compound of Example 2 in 25 ml of methylene chloride is added dropwise to a solution of 1.79 grams of meta-chloroperoxybenzoic acid (m-CpBA) in methylene chloride. After stirring the reaction mixture at room temperature overnight under $N_2$, the solvent is removed under reduced pressure. The resulting residue is dissolved in methylene chloride and successively washed with saturated aqueous $NaHSO_3$, saturated aqueous $NAHCO_3$, brine and dried and evaporated. The resulting residue is recrystallized from isopropanol to afford the title compound as a white crystalline solid: m.p. 72 - 74 °C.
Anal. Calcd. for $C_{40}H_{79}NO_5$: C, 73.5; H, 12.2; N, 2.1.
Found: C, 73.1; H, 12.4; N, 2.0.

Example 4: N,N-Bis[2-acetoxyethyl]-N-[2′-methoxycarbonylethyl]amine

The procedure of Example 2 is repeated using 5.0 grams of N-[2-methoxycarbonylethyl]diethanolamine, 3.7 ml of acetyl chloride and 7.3 ml of triethylamine in methylene chloride to afford the title compound as a yellow oil.

Example 5: N,N-Bis[2-acetoxyethyl]hydroxylamine

The procedure of Example 3 is repeated using 1.47 grams of the compound of Example 4 and 0.92 grams of m-CpBA in methylene chloride, to afford the title compound as a light yellow oil.

Example 6: N,N-Bis[2-(3′,5′-di-tert-butyl-4′-hydroxyphenylpropionyloxy)ethyl]-N-[2″-methoxycarbonylethyl]-amine

The procedure of Example 2 is repeated using 2.65 grams of N-(2-methoxycarbonylethyl)-diethanolamine, 7.85 grams of 2-(3′,5′-di-tert-butyl-4-hydroxyphenyl)propionyl chloride and 3.9 ml of triethylamine in methylene chloride to afford the title compound as a light yellow oil.
Anal. Calcd. for $C_{42}H_{65}NO_8$: C, 70.9; H, 9.2; N, 2.0.
Found: C, 70.1; H, 9.3; N, 2.1.

Example 7: N,N-Bis[2-(3′,5′-di-tert-butyl-4′-hydroxyphenylpropionyloxy)ethyl]hydroxylamine

The procedure of Example 3 is repeated using 6.05 grams of the compound of Example 6 and 1.98 grams of m-CpBA in methylene chloride, to afford the title compound as a white solid: m.p. 107 - 110 °C.
Anal. Calcd. for $C_{38}H_{69}NO_7$: C, 71.1; H, 9.3; N, 2.2.
Found: C, 71.3; H, 9.5; N, 2.2.

Example 8: N,N-Bis[2-(3',5'-di-tert-butyl-4'-hydroxybenzoyloxy)ethyl]-N-[2''-methoxycarbonylethyl]amine

Procedure of Example 2 is repeated using 5.37 grams of N-(2-methoxycarbonylethyl)diethanolamine, 10.75 grams of 3,5-di-tert-butyl-4-hydroxybenzoylchloride and 5.58 ml of triethylamine in methylene chloride to afford the title compound as a white foam.
Anal. Calcd. for $C_{38}H_{57}NO_8$: C, 69.6; H, 8.8; N, 2.1.
Found: C, 69.9; H, 8.7; N, 1.9.

Example 9: 1N,N-Bis[2-(3',5'-di-tert-butyl-4'-hydroxybenzoyloxy)ethyl]hydroxylamine

The procedure of Example 3 is repeated using 9.32 grams of the compound of Example 8 and 3.06 grams of m-CpBA in methylene chloride to afford the title compound as a white solid: m.p. 151 - 153 °C.
Anal. Calcd. for $C_{34}H_{51}NO_7$: C, 69.7; H, 8.8; N, 2.4.
Found: C, 70.1; H, 9.2; N, 2.4.

Example 10: Processing of Polypropylene

Base Formulation

| | |
|---|---|
| Polypropylene* | 100 parts |
| Calcium Stearate | 0.10 part |

*®Profax 6501 from Himont U.S.A.

Stabilizers are solvent blended into polypropylene as solutions in methylene chloride and after removal of solvent by evaporation at reduced pressure, the resins are extruded using the following extruder conditions:

| | | Temperature (°C) |
|---|---|---|
| Cylinder #1 | | 232 |
| Cylinder #2 | | 246 |
| Cylinder #3 | | 260 |
| Gate #1 | | 260 |
| Gate #2 | | 260 |
| Gate #3 | | 260 |
| RPM | 100 | |

The melt flow rate (MFR) is determined by ASTM method 1238 condition L. The melt flow rate is the measure of the molecular weight for a specific type of polymer. Higher values mean lower molecular weight and indicate decompositions of the polymer. The results are shown in Table 1.

Table 1:

| Additive | MFR After Extrusion (g/10 min) 1 | 5 |
|---|---|---|
| Base Resin | 7.9 | 44.3 |
| 0.1 % Antioxidant A | 5.3 | 8.7 |
| 0.1 % Antioxidant A + 0.05 % of compound of Ex. 3 | 2.8 | 5.7 |

Antioxidant A: neopentyl tetrakis[3-(3',5'-di-tert-butyl-4'-hydroxyphenyl)propionate]

Example 11: Stabilization of Polypropylene

Unstabilized polypropylene powder (®Hercules Profax 6501) is thoroughly blended with the indicated amount of additive. The blended materials are then milled on a two-roll mill at 182 °C for five minutes, after which time the stabilized polypropylene is sheeted from the mill and allowed to cool. The milled polypropylene is then cut into pieces and compression molded on a hydraulic press at 250 °C and 1.2 x $10^6$ Pa into 0.635 mm plaques. The samples are exposed in a fluorescent sunlight/black light chamber until failure. Failure is taken as the hours required to reach 0.5 carbonyl absorbance by infrared spectroscopy on the exposed films.

Table 2:

| Additive | FS/BL Test Results (Hours to Failure) |
|---|---|
| None | 100 |
| 0.2 % of compound of Ex. 3 | 370 |

The instant compounds are thus seen to be effective color and process stabilizers in polypropylene compositions.

**Claims**

**1.** A compound of the formula I

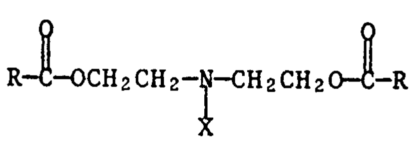

(I)

wherein each R is alkyl of 1 to 36 carbon atoms, cycloalkyl of 5 to 12 carbon atoms, phenyl, phenyl substituted by alkyl of 1 to 36 carbon atoms, aralkyl of 7 to 9 carbon atoms, said aralkyl substituted by

alkyl of 1 to 36 carbon atoms, or is

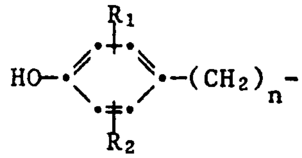

(II)

wherein $R_1$ and $R_2$ are independently alkyl of 1 to 18 carbon atoms, cycloalkyl of 5 to 12 carbon atoms, phenyl or aralkyl of 7 to 9 carbon atoms, and n is 0, 1 or 2; and
X is hydroxyl or

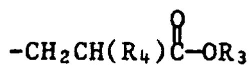

wherein $R_3$ is alkyl of 1 to 36 carbon atoms, cycloalkyl of 5 to 12 carbon atoms, phenyl, phenyl substituted by alkyl of 1 to 36 carbon atoms, aralkyl of 7 to 9 carbon atoms or said aralkyl substituted by alkyl of 1 to 36 carbon atoms, and $R_4$ is hydrogen or methyl.

**2.** The compound of claim 1, wherein the radicals R are independently alkyl of 1 to 18 carbon atoms, cyclopentyl, cyclohexyl, phenyl, benzyl, $\alpha$-methylbenzyl or $\alpha,\alpha$-dimethylbenzyl.

**3.** The compound of claim 1, wherein R is a group of the formula II with $R_1$, $R_2$ and n being as defined in claim 1.

**4.** The compound of claim 3, wherein R is a group of the formula IIa

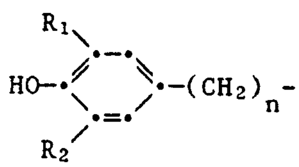

(IIa)

and $R_1$, $R_2$ and n are as defined in claim 1.

**5.** The compound of claim 3, wherein $R_1$ and $R_2$ are independently alkyl of 4 to 8 carbon atoms.

**6.** The compound of claim 1, wherein X is hydroxyl.

**7.** The compound of claim 1, wherein X is

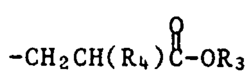

and $R_3$ and $R_4$ are as defined in claim 1.

**8.** The compounds
N,N-bis[2-stearoyloxyethyl]-N-[2′-methoxycarbonylethyl]amine,
N,N-bis[2-stearoylethyl]hydroxylamine,
N,N-bis[2-acetoxyethyl]-N-[2′-methoxycarbonylethyl]amine,
N,N-bis[2-acetoxyethyl]hydroxylamine,
N,N-bis[2-(3′,5′-di-tert-butyl-4′-hydroxyphenylpropionyloxy)ethyl]-N-[2″-methoxycarbonylethyl]amine,
N,N-bis[2-(3′,5′-di-tert-butyl-4′-hydroxyphenylpropionyloxy)ethyl]hydroxylamine,
N,N-bis[2-(3′,5′-di-tert-butyl-4′-hydroxybenzoyloxy)ethyl]-N-[2″-methoxycarbonylethyl]amine and

N,N-bis[2-(3′,5′-di-tert-butyl-4′-hydroxybenzoyloxy)ethyl]hydroxylamine according to claim 1.

**9.** A composition of matter comprising an organic material subject to oxidative, thermal and actinic degradation and at least one compound of claim 1.

**10.** The composition of claim 9, wherein the organic material is a synthetic polymer.

**11.** The composition of claim 10, wherein the synthetic polymer is a polyolefin homopolymer or copolymer.

**12.** The composition of claim 9, which also contains a metal salt of a higher fatty acid and/or a phenolic antioxidant.

**13.** The composition of claim 12, wherein said phenolic antioxidant in neopentanetetrayl tetrakis(3,5-di-tert-butyl-4-hydroxyhydrocinnamate),n-octadecyl 3,5-di-tert-butyl-4-hydroxyhydrocinnamate, 1,3,5-trimethyl-2,4,6-tris(3,5-di-tert-butyl-4-hydroxybenzyl)benzene, 1,3,5-tris(3,5-di-tert-butyl-4-hydroxybenzyl)-isocyanurate, 2,6-di-tert-butyl-p-cresol or 2,2′-ethylidene-bis(4,6-di-tert-butylphenol).

**14.** The composition of claim 9, which also contains antioxidants, benzotriazole UV absorbers, hindered amine light stabilizers, phosphites, thiosynergists or mixtures thereof.

**15.** Use of compounds of formula I according to claim 1 for stabilizing an organic material against oxidative, thermal and actinic degradation.

## Patentansprüche

**1.** Verbindungen der Formel I

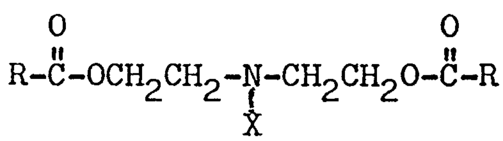

(I)

worin jedes R Alkyl mit 1 bis 36 Kohlenstoffatomen, Cycloalkyl mit 5 bis 12 Kohlenstoffatomen, Phenyl, Phenyl, substituiert durch Alkyl mit 1 bis 36 Kohlenstoffatomen, Aralkyl mit 7 bis 9 Kohlenstoffatomen, dieses Aralkyl, substituiert durch Alkyl mit 1 bis 36 Kohlenstoffatomen, bedeutet oder für

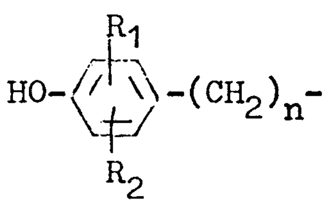

(II)

steht, worin $R_1$ und $R_2$ unabhängig Alkyl mit 1 bis 18 Kohlenstoffatomen, Cycloalkyl mit 5 bis 12 Kohlenstoffatomen, Phenyl oder Aralkyl mit 7 bis 9 Kohlenstoffatomen bedeuten und n für 0, 1 oder 2 steht; und X Hydroxyl oder

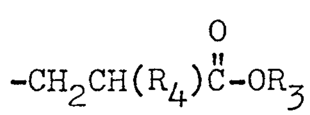

bedeutet, wobei $R_3$ Alkyl mit 1 bis 36 Kohlenstoffatomen, Cycloalkyl mit 5 bis 12 Kohlenstoffatomen, Phenyl, Phenyl, substituiert durch Alkyl mit 1 bis 36 Kohlenstoffatomen, Aralkyl mit 7 bis 9 Kohlenstoffatomen oder dieses Aralkyl, substituiert durch Alkyl mit 1 bis 36 Kohlenstoffatomen, bedeutet und $R_4$ für Wasserstoff oder Methyl steht.

**2.** Verbindungen gemäß Anspruch 1, worin die Reste R unabhängig Alkyl mit 1 bis 18 Kohlenstoffatomen, Cyclopentyl, Cyclohexyl, Phenyl, Benzyl, α-Methylbenzyl oder α,α-Dimethylbenzyl bedeuten.

**3.** Verbindungen gemäß Anspruch 1, worin R für eine Gruppe der Formel II steht, wobei $R_1$, $R_2$ und n die in Anspruch 1 angegebene Bedeutung besitzen.

**4.** Verbindungen gemäß Anspruch 3, worin R für eine Gruppe der Formel IIa

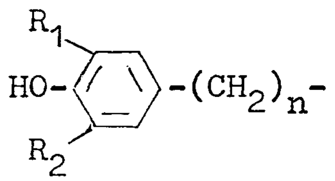

(IIa)

steht und $R_1$, $R_2$ und n wie in Anspruch 1 definiert sind.

**5.** Verbindungen gemäß Anspruch 3, worin $R_1$ und $R_2$ unabhängig Alkyl mit 4 bis 8 Kohlenstoffatomen bedeuten.

**6.** Verbindungen gemäß Anspruch 1, worin X für Hydroxyl steht.

**7.** Verbindungen gemäß Anspruch 1, worin X für

$$-CH_2CH(R_4)\overset{O}{\overset{\|}{C}}-OR_3$$

steht und $R_3$ und $R_4$ wie in Anspruch 1 definiert sind.

**8.** Die Verbindungen
N,N-Bis-(2-stearoyloxyethyl)-N-(2'-methoxycarbonylethyl)-amin,
N,N-Bis-(2-stearoyloxyethyl)-hydroxylamin,
N,N-Bis-(2-acetoxyethyl)-N-(2'-methoxycarbonylethyl)-amin,
N,N-Bis-(2-acetoxyethyl)-hydroxylamin,
N,N-Bis-[2-(3',5'-di-tert.-butyl-4'-hydroxyphenylpropionyloxy)-ethyl]-N-(2"-methoxycarbonylethyl)-amin,
N,N-Bis-[2-(3',5'-di-tert.-butyl-4'-hydroxyphenylpropionyloxy)-ethyl]-hydroxylamin,
N,N-Bis-[2-(3',5'-di-tert.-butyl-4'-hydroxybenzoyloxy)-ethyl]-N-(2"-methoxycarbonylethyl)-amin und
N,N-Bis-[2-(3',5'-di-tert.-butyl-4'-hydroxybenzoyloxy)-ethyl]-hydroxylamin gemäß Anspruch 1.

**9.** Materialzusammensetzung, enthaltend ein organisches Material, das einem oxidativen, thermischen und strahlungsbedingten Abbau unterliegt, und zumindest eine Verbindung gemäß Anspruch 1.

**10.** Zusammensetzung gemäß Anspruch 9, worin das organische Material ein synthetisches Polymeres ist.

**11.** Zusammensetzung gemäß Anspruch 10, worin das synthetische Polymere ein Polyolefinhomopolymers oder -copolymeres ist.

**12.** Zusammensetzung gemäß Anspruch 9, die auch ein Metallsalz einer höheren Fettsäure und/oder ein phenolisches Antioxidans enthält.

**13.** Zusammensetzung gemäß Anspruch 12, worin das phenolische Antioxidans Neopentantetrayl-tetrakis-(3,5-di-tert.-butyl-4-hydroxyhydrocinnamat), n-Octadecyl-3,5-di-tert.-butyl-4-hydroxyhydrocinnamat, 1,3,5-Trimethyl-2,4,6-tris-(3,5-di-tert.-butyl-4-hydroxybenzyl)-benzol, 1,3,5-Tris-(3,5-di-tert.-butyl-4-hydroxybenzyl)-isocyanurat, 2,6-Di-tert.-butyl-p-cresol oder 2,2'-Ethyliden-bis-(4,6-di-tert.-butylphenol) ist.

**14.** Zusammensetzung gemäß Anspruch 9, die auch Antioxidantien, Benzotriazol-UV-Absorber, gehinderte Aminlichtstabilisatoren, Phosphite, Thiosynergisten oder Mischungen hiervon enthält.

**15.** Verwendung der Verbindungen der Formel I gemäß Anspruch 1 zur Stabilisierung eines organischen Materials gegenüber oxidativem, thermischen und strahlungsbedingtem Abbau.

**Revendications**

**1.** Un composé répondant à la formule I

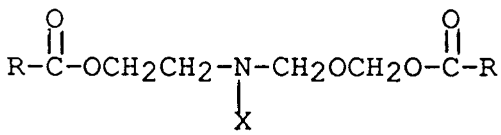

(I)

dans laquelle chaque R représente un radical alkyle de 1 à 36 atomes de carbone, cycloalkyle de 5 à 12 atomes de carbone, phényle, phényle avec un substituant alkyle de 1 à 36 atomes de carbone, aralkyle de 7 à 9 atomes de carbone, ledit aralkyle substitué par un groupement alkyle de 1 à 36 atomes de carbones, ou représente un radical répondant à la formule :

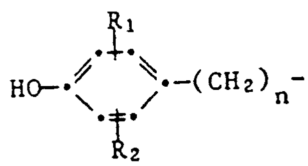

(II)

dans laquelle $R_1$ et $R_2$ sont chacun indépendamment un groupement alkyle de 1 à 18 atomes de carbone, cycloalkyle de 5 à 12 atomes de carbone, phényle ou aralkyle de 7 à 9 atomes de carbone, et n vaut 0, 1 ou 2 ; et
X représente un groupement hydroxyle ou

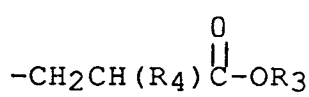

dans lequel $R_3$ est un groupement alkyle de 1 à 36 atomes de carbone, cycloalkyle de 5 à 12 atomes de carbone, phényle, phényle avec un substituant alkyle de 1 à 36 atomes de carbone, aralkyle de 7 à 9 atomes de carbone ou un aralkyle de ce genre porteur d'un substituant alkyle de 1 à 36 atomes de carbones, et $R_4$ est un atome d'hydrogène ou un groupe méthyle.

**2.** Le composé de la revendication 1 dans lequel les radicaux R sont indépendamment un groupement alkyle de 1 à 18 atomes de carbone, cyclopentyle, cyclohexyle, phényle, benzyle, a-méthylbenzyle ou a,a-diméthylbenzyle.

**3.** Le composé de la revendication 1 dans lequel R est un groupe de formule II avec $R_1$, $R_2$ et n définis comme à la revendication 1.

**4.** Le composé de la revendication 3 dans lequel R est un groupe de la formule IIa :

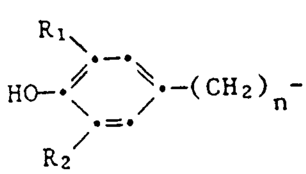

(IIa)

et $R_1$, $R_2$ et n sont comme définis à la revendication 1.

**5.** Le composé de la revendication 3 dans lequel $R_1$ et $R_2$ sont indépendamment un groupement alkyle de 4 à 8 atomes de carbone.

**6.** Le composé de la revendication 1 dans lequel X est un groupement hydroxyle.

**7.** Le composé de la revendication 1 dans lequel X est

$$-CH_2CH(R_4)\overset{O}{\overset{\|}{C}}-OR_3$$

et $R_3$ et $R_4$ sont comme définis à la revendication 1.

**8.** Les composés
N,N-bis[2-stéaroyleoxyéthyl]-N-[2'-méthoxycarbonyléthyl]amine,
N,N-bis[2-stéaroyleoxyéthyl]hydroxylamine,
N,N-bis[2-acétoxyéthyl]-N-[2'-méthoxycarbonyléthyl]amine,
N,N-bis[2-acétoxyéthyl]hydroxylamine,
N,N-bis[2-(3',5'-di-tert-butyl-4'-hydroxyphénylpropionyloxy)-éthyl]-N-[2"-méthoxycarbonyléthyl]amine,
N,N-bis[2-(3',5'-di-tert-butyl-4'-hydroxyphénylpropionyloxy)-éthyl]hydroxylamine,
N,N-bis[2-(3',5'-di-tert-butyl-4'-hydroxybenzoyloxy)éthyl]-N-[2"-méthoxycarbonyléthyl]amine et
N,N-bis[2-(3',5'-di-tert-butyl-4'-hydroxybenzoyloxy)éthyl]-hydroxylamine selon la revendication 1.

**9.** Une composition comprenant un matériau organique sujet à dégradation oxydative, thermique et actinique et au moins un composé de la revendication 1.

**10.** La composition de la revendication 9 dans laquelle le matériau organique est un polymère synthétique.

**11.** La composition de la revendication 10 dans laquelle le polymère synthétique est un homopolymère ou un copolymère de polyoléfines.

**12.** La composition de la revendication 9 qui contient aussi un sel métallique d'un acide gras supérieur et/ou un antioxydant phénolique.

**13.** La composition de la revendication 12 dans laquelle ledit antioxydant phénolique est le tétrakis(3,5-di-tert-butyl-4-hydroxyhydrocinnamate) de néopentanetétrayle, le 3,5-di-tert-butyl-4-hydroxyhydrocinnamate de n-octadécyle, le 1,3,5-triméthyl-2,4,6-tris(3,5-di-tert-butyl-4-hydroxy-benzyl)benzène, l'isocyanurate de tris(3,5-di-tert-butyl-4-hydroxy-benzyle), le 2,6-di-tert-butyl-p-crésol ou le 2,2'-éthylidène-bis (4,6-di-tert-butylphénol).

**14.** La composition de la revendication 9 qui contient aussi des antioxydants, des absorbeurs d'UV à base de benzotriazole, des stabilisants à la lumière à base d'amines à empêchement stérique, des phosphites, des thiosynergistes ou des mélanges de ceux-ci.

**15.** L'utilisation de composés de formule I selon la revendication 1 pour stabiliser un matériau organique vis-à-vis des degradations actinique, thermique et par oxydation.